# EUROPEAN PATENT APPLICATION

(11) **EP 1 275 721 A1**
(43) Date of publication of application: **15.01.2003**
(21) Application number: 01919887.8
(22) Date of filing: 11.04.2001
(51) Int. Cl.: C12N 15/54, C12N 9/10, C12N 1/21, C12P 19/26, C07H 15/12

(54) **ALPHA 1,4-N-ACETYLGALACTOSAMINE TRANSFERASE GENE AND PROCESS FOR PRODUCING THIS ENZYME AND N-ACETYLGALACTOSAMINE-CONTAINING COMPLEX CARBOHYDRATE**

(30) Priority: 11.04.2000 JP 2000109150
(71) Applicant: KYOWA HAKKO KOGYO CO., LTD., Chiyoda-ku, Tokyo 100-8185 (JP)
(72) Inventor: ENDO, Tetsuo, Tokyo Research Lab., Machida-shi, Tokyo 194-8533 (JP); KAKITA, Shingo, Tokyo Research Lab., Machida-shi, Tokyo 194-8533 (JP); KOIZUMI, Satoshi, Tokyo Research Lab., Machida-shi, Tokyo 194-8533 (JP); OZAKI, Akio, Technical Research Lab., Hofu-shi, Yamaguchi 747-8522 (JP)
(74) Representative: VOSSIUS & PARTNER
(86) International application number: JP0103111
(87) International publication number: WO01077337

(57) **Abstract**

According to the present invention, α1,4-GalNAc transferase can be produced in a large amount by using a microorganism. Also, a GalNAc-containing complex carbohydrate can be economically produced in a large amount by contacting a microorganism expressing the transferase, UDP-GalNAc and an acceptor complex carbohydrate in an aqueous medium.

## Description

### TECHNICAL FIELD

The present invention relates to a protein having α1,4-GalNAc transferase activity, a DNA encoding the protein, a recombinant DNA comprising the DNA, a transformant comprising the recombinant DNA, a process for producing α1,4-GalNAc transferase and a GalNAc-containing complex carbohydrate using the transformant.

### BACKGROUND OF THE INVENTION

The importance of complex carbohydrates existing in the living body has been reported on various complex carbohydrates [*Essentials of Glycobiology,* Cold Spring Harbor Press (1999)], and it is expected to develop complex carbohydrates into medicaments effective for the infection control of bacteria, viruses and the like, application to cardiovascular disorders, immunotherapy and the like.

Regarding GalNAc-containing complex carbohydrates, since an α1,4-GalNAc transferase gene derived from animal involved in the synthesis of a GalNAc-containing complex carbohydrate has high homology with a tumor suppressor gene, it is expected that a complex carbohydrate produced by the enzyme will show antitumor activity and its use as a diagnostic agent in the field of cancers can also be considered, but industrial production methods of GalNAc-containing complex carbohydrates are not known.

As the α1,4-GalNAc transferase relating to the biosynthesis of GalNAc-containing complex carbohydrates, enzymes derived from animal [*J. Biol. Chem.,* 270, 22190 (1995), *Glycobiology*, 9, 697 (1999), *J. Biol. Chem.*, 274, 13933 (1999)] are known, and a gene encoding the enzyme has also been obtained [*J. Biol. Chem*., 274, 13933 (1999)]. The enzyme uses a complex carbohydrate having glucuronic acid at the non-reducing terminal as its substrate, but there is no report that the enzyme uses as its substrate a complex carbohydrate having galactose or GalNAc at the non-reducing terminal.

On the other hand, in the case of a procaryote *Campylobacter jejuni*, it is known that it has a ganglioside structure in the lipopolysaccharide of its cell surface layer *[J. Bacteriol*., 174, 1324 (1992), *Biochemistry*, 33, 241 (1994), *Biochemistry*, 33, 250 (1994), *Infect. Immun*., 62, 2122 (1994)], and a β1,4-GalNAc transferase gene relating to its biosynthesis has been obtained [*J. Biol. Chem.*, 275, 3896 (2000)]. Also, a sugar chain structure in which GalNAc is linked to GalNAc by α1,4-linkage is known in the microorganism [*Crit. Rev. Microbiol.*, 22, 139 (1996)]. In addition, it is considered that the wlaC gene comprised in the gene cluster relating to the biosynthesis of lipopolysaccharides may be the gene which encodes galactose or GalNAc transferase, but its function has not been brought out yet *[Microbiology,* 144, 2049 (1998)].

### DISCLOSURE OF THE INVENTION

An object of the present invention is to provide a protein having α1,4-GalNAc transferase activity, a DNA encoding the protein, a process for producing the protein having α1,4-GalNAc transferase activity using the DNA and an industrial process for producing a GalNAc-containing complex carbohydrate using the protein.

In order to solve the above problems, the present inventors have conducted intensive studies and succeeded in isolating a DNA encoding α1,4-GalNAc transferase from a microorganism belonging to the genus *Campylobacter.* Thereafter, the present invention has been accomplished by further finding that α1,4-GalNAc transferase and a GalNAc-containing complex carbohydrate can be produced in a large amount using the DNA.

Accordingly, the present invention relates to the following items (1) to (20).
(1) A process for producing a complex carbohydrate containing N-acetylgalactosamine (hereinafter referred to as "GalNAc"), which comprises selecting, as an enzyme source, a culture of a transformant expressing a protein having al,4-N-acetylgalactosamine transferase (hereinafter referred to as "α1,4-GalNAc transferase") activity derived from a microorganism or a treated product of the culture; allowing the enzyme source, an acceptor complex carbohydrate and uridine diphospho-N-acetylgalactosamine (hereinafter referred to as "UDP-GalNAc") to be present in an aqueous medium; transferring GalNAc to the acceptor complex carbohydrate to form and accumulate the GalNAc-containing complex carbohydrate in the aqueous medium; and recovering the GalNAc-containing complex carbohydrate from the aqueous medium.
(2) A process for producing a protein having α1,4-GalNAc transferase activity, which comprises culturing a transformant expressing a protein having α1,4-GalNAc transferase activity derived from a microorganism in a medium; allowing the protein having α1,4-GalNAc transferase activity to form and accumulate in the culture; and recovering the protein from the culture.
(3) The process according to (1), wherein the GalNAc-containing complex carbohydrate is NOS-α having a GalNAcα1-4Galβ1-4GlcNAcβ-1-3Galβ1-4Glc structure or NOS-β having a GalNAcα1-4Galβ1-4Glc structure.
(4) The process according to (1) or (2), wherein the protein having α1,4-GalNAc transferase activity derived from a microorganism is a protein selected from proteins described in the following [1] to [3]:
   [1] a protein having the transferase activity derived from a microorganism belonging to the genus *Campylobacter;*
   [2] a protein comprising the amino acid sequence represented by SEQ ID NO:1;
   [3] a protein which comprises an amino acid sequence in which at least one amino acid in the amino acid sequence represented by SEQ ID NO:1 is deleted, substituted or added and has the transferase activity.
(5) The process according to (1) or (2), wherein the transformant is a transformant obtained by introducing a recombinant DNA into a microorganism.
(6) The process according to (5), wherein the microorganism is a microorganism belonging to the genus *Escherichia*.
(7) The process according to (6), wherein the microorganism belonging to the genus *Escherichia* is *Escherichia coli.*
(8) The process according to (5), wherein the recombinant DNA is a recombinant DNA containing a DNA selected from DNAs described in the following [1] to [6]:
   [1] a DNA encoding a protein having α1,4-GalNAc transferase activity derived from a microorganism;
   [2] a DNA encoding a protein having α1,4-GalNAc transferase activity derived from a microorganism belonging to the genus *Campylobacter*;
   [3] a DNA encoding a protein comprising the amino acid sequence represented by SEQ ID NO:1;
   [4] a DNA comprising the nucleotide sequence represented by SEQ ID NO:2;
   [5] a DNA encoding a protein which comprises an amino acid sequence in which at least one amino acid in the amino acid sequence represented by SEQ ID NO:1 is deleted, substituted or added, and has α1,4-GalNAc transferase activity;
   [6] a DNA which hybridizes with the DNA of any one of above [1] to [5] under stringent conditions and encodes a protein having α1,4-GalNAc transferase activity.
(9) The process for producing GalNAc-containing complex carbohydrate according to (1), wherein the acceptor complex carbohydrate is a complex carbohydrate which contains an oligosaccharide consisting of 10 or less sugar having galactose or GalNAc in the non-reducing terminal.
(10) The process according to (9), wherein the oligosaccharide has a lactose, N-acetyllactosamine, lacto-N-neotetraose, lacto-N-tetraose, paralacto-N-neohexaose, Le^{x} or Le^{a} structure in the non-reducing terminal.
(11) The process for producing GalNAc-containing complex carbohydrate according to (1), wherein the treated product of the culture is a concentrated product of the culture, a dried product of the culture, cells obtained by centrifuging the culture, a dried product of the cells, a freeze-dried product of the cells, a surfactant-treated product of the cells, an ultrasonic-treated product of the cells, a mechanically ground product of the cells, a solvent-treated product of the cells, an enzyme-treated product of the cells, a protein fraction of the cells, an immobilized product of the cells or an enzyme preparation obtained by extracting the cells.
(12) A GalNAc-containing complex carbohydrate according to the following [1] or [2]:
   [1] NOS-α having a GalNAcαl-4Galβ1-4GlcNAcβ-1-3Galβ1-4Glc structure;
   [2] NOS-β having a GalNAcαl-4Galβ1-4Glc structure.
(13) An agent for synthesizing a GalNAc-containing complex carbohydrate, which contains a protein comprising the amino acid sequence represented by SEQ ID NO:1.
(14) A protein which comprises an amino acid sequence in which at least one amino acid in the amino acid sequence represented by SEQ ID NO:1 is deleted, substituted or added, and has α1,4-GalNAc transferase activity.
(15) A DNA according to any one of the following
   [1] to [3]:

   [1] a DNA encoding the protein according to (14);
   [2] a DNA comprising a nucleotide sequence in which at least one base in the nucleotide sequence represented by SEQ ID NO:2 is deleted, substituted or added, which is a DNA encoding a protein having α1,4-GalNAc transferase activity;
   [3] a DNA which hybridizes with any one of DNAs selected from the DNA of [1], the DNA of [2] and a DNA comprising the nucleotide sequence represented by SEQ ID NO:2 under stringent conditions, and encodes a protein having α1,4-GalNAc transferase activity.
(16) A recombinant DNA which is obtained by ligating the DNA according to (15) with a vector.
(17) A transformant which is obtained by introducing the recombinant DNA according to (16) into a host cell.
(18) The transformant according to (17), wherein the host cell is a microorganism.
(19) The transformant according to (18), wherein the microorganism is a microorganism belonging to the genus *Escherichia*.
(20) The transformant according to (19), wherein the microorganism belonging to the genus *Escherichia* is *Escherichia coli.*

The present invention is described below in detail.

A protein having α1,4-GalNAc transferase activity as the protein of the present invention may be any protein derived from a microorganism and having the enzyme activity, but is preferably a protein derived from a microorganism belonging to the genus *Campylobacter,* more preferably a protein derived from *Campylobacter jejuni.* Examples include a protein comprising the amino acid sequence represented by SEQ ID NO:1, and a protein which comprises an amino acid sequence in which at least one amino acid in the amino acid sequence represented by SEQ ID NO:1 is deleted, substituted or added and has α1,4-GalNAc transferase activity.

The protein comprising the amino acid sequence in which at least one amino acid is deleted, substituted or added and has α1,4-GalNAc transferase activity can be obtained by introducing site-specific mutation into, e.g., a DNA encoding a protein comprising the amino acid sequence represented by SEQ ID NO:1, by using a site-directed mutagenesis described in *Molecular Cloning, A Laboratory Manual,* Second Edition, Cold Spring Harbor Laboratory Press (1989) (hereinafter referred to as *"Molecular Cloning,* Second Edition"); *Current Protocols in Molecular Biology,* John Wiley & Sons (1987-1997) (hereinafter referred to as *"Current Protocols in Molecular Biology"); Nucleic Acids Res.,* 10, 6487 (1982); *Proc. Natl. Acad. Sci., USA*, 79, 6409 (1982); *Gene*, 34, 315 (1985); *Nucleic Acids Res.*, 13, 4431 (1985); *Proc. Natl. Acad. Sci., USA*, 82, 488 (1985) and the like.

The number of amino acids to be deleted, substituted or added is not particularly limited but is such a number that deletion, substitution or addition can be carried out by well known methods such as the site-directed mutagenesis and the like, which is from 1 to several tens, preferably from 1 to 20, more preferably from 1 to 10, and most preferably from 1 to 5.

Also, in order to keep the α1,4-GalNAc transferase activity of the protein of the present invention, it is preferable that it has at least 60% or more, generally 80% or more, particularly 95% or more, of identity with the amino acid sequence represented by SEQ ID NO:1, when calculated using BLAST [*J. Mol. Biol.*, 215, 403 (1990)], FASTA *[Methods in Enzymology,* 183, 63 (1990)] or the like.

The DNA encoding a protein having α1,4-GalNAc transferase activity used in the production processes of the present invention may be any DNA encoding a protein having the transferase activity derived from a microorganism, but is preferably a DNA derived from a microorganism belonging to the genus *Campylobacter,* more preferably a DNA derived from *Campylobacter jejuni.* Examples include:
(1) a DNA encoding a protein comprising the amino acid sequence represented by SEQ ID NO:1;
(2) a DNA comprising the nucleotide sequence represented by SEQ ID NO:2;
(3) a DNA encoding a protein which comprises an amino acid sequence in which at least one amino acid in the amino acid sequence represented by SEQ ID NO:1 is deleted, substituted or added, and has α1,4-GalNAc transferase activity; and
(4) a DNA which hybridizes with any one of the DNAs described in the above (1) to (3) under stringent conditions and encodes a protein having α1,4-GalNAc transferase activity.

The above DNA which is hybridizable under stringent conditions is a DNA obtained by colony hybridization, plaque hybridization, Southern blot hybridization or the like using a part or a whole of the DNA in any one of the above (1) to (3) as the probe, and specific examples of the DNA include those which can be identified by carrying out hybridization at 65°C in the presence of 0.7 to 1.0 mol/l of sodium chloride using a filter on which colony- or plaque-derived DNAs are immobilized and then washing the filter under conditions of 65°C using 0.1× to 2× SSC solution (1× SSC contains 150 mmol/l sodium chloride and 15 mmol/l sodium citrate). The hybridization can be carried out in accordance with the methods described in *Molecular Cloning,* Second Edition, *Current Protocols in Molecular Biology, DNA Cloning 1: Core Techniques, A Practical* *Approach,* Second Edition, Oxford University (1995) and the like. Examples include a DNA having at least 60% or more, preferably a DNA having 80% or more, more preferably a DNA having 95% or more, of identity, with the nucleotide sequence represented by SEQ ID NO:2, when calculated using BLAST [*J. Mol. Biol*., 215, 403 (1990)], FASTA *[Methods* in *Enzymology,* 183, 63 (1990)] or the like.

An example of the process for producing the protein having α1,4-GalNAc transferase activity is a process in which a recombinant DNA is prepared by ligating the DNA encoding the protein prepared by the above process with a vector DNA in accordance with the method described in *Molecular Cloning,* Second Edition, a transformant is prepared by transforming a host cell with the recombinant DNA in accordance with the method described in *Molecular Cloning,* Second Edition, the transformant is cultured in an appropriate medium in which the transformant can grow to thereby accumulate the protein in the culture, and then the protein is recovered from the culture. In addition, an example of the method for recovering the protein from the culture is a method in which the protein is solubilized and then isolated and purified by ion exchange, gel filtration or hydrophobic chromatography or the like, or by a combination of the chromatography.

The GalNAc-containing complex carbohydrate of the present invention can be obtained by using a culture of a transformant capable of producing the protein, a treated product of the culture or purified product of the protein as the enzyme source, and allowing it to coexist with an acceptor complex carbohydrate and UDP-GalNAc in an aqueous solvent.

The acceptor complex carbohydrate is not particularly limited, so long as it can be used as a substrate of the protein having α1,4-GalNAc transferase activity encoded by the DNA of the present invention, but a saccharide having galactose or GalNAc in its non-reducing terminal is exemplified as a preferable substrate, and an oligosaccharide having a lactose, N-acetyllactosamine, lacto-N-neotetraose, lacto-N-tetraose, Le^{x} or Le^{a} structure in its non-reducing terminal is exemplified as a more preferable substrate.

The thus formed GalNAc-containing complex carbohydrate can be recovered by general chromatography using activated charcoal, an ion exchange resin or the like.

The present invention is explained below in detail.

### [1] Production of a transformant expressing a protein having α1,4-GalNAc transferase activity

### (1) Preparation of the DNA encoding a protein having α1,4-GalNac transferase activity

The DNA encoding a protein having α1,4-GalNAc transferase activity can be prepared using any microorganism, so long as the microorganism has the enzyme activity. Preferred examples include a microorganism belonging to the genus *Campylobacter* and the like, and more preferred examples include *Campylobacter jejuni* (ATCC 43446) and the like.

The microorganism belonging to the genus *Campylobacter* is cultured by a known method [e.g., *Microbiology*, 144, 2049 (1998)]. After culturing, chromosomal DNA of the microorganism is isolated and purified by a known method (e.g., method described in *Current Protocols in Molecular Biology*).

A fragment containing the DNA encoding a protein having α1,4-GalNAc transferase activity can be obtained by PCR [e.g., *PCR Protocols,* Humana Press (1993)] using a primer set prepared upon known nucleotide sequences *[Microbiology,* 144, 2049 (1998)] and the chromosomal DNA as the template.

Also, the desired DNA can be obtained by a hybridization method using a synthetic DNA designed by known sequences as a probe, or the like.

Furthermore, the DNA encoding a protein having α1,4-GalNAc transferase activity can be artificially synthesized by PCR or the like using a synthetic DNA according to the usual method [e.g., *PCR Protocols,* Humana Press (1993), *etc*.].

### (2) Cloning of DNA encoding a protein having α1,4-GalNAc transferase activity and confirmation of its nucleotide sequence

The DNA prepared in the above (1) is ligated with a vector according to the usual method as it is or after digestion with an appropriate restriction enzyme.

The vector with which the DNA is ligated may be any vector, such as a phage vector, a plasmid vector or the like, so long as it can replicate autonomously in *Escherichia coli* K12. Examples include ZAP Express [manufactured by Stratagene, *Strategies*, 5, 58 (1992)], pBluescript II SK(+) [manufactured by Stratagene, *Nucleic Acids* Res., 17, 9494 (1989)], λzap II (manufactured by Stratagene), λgt10 and λgt11 [*DNA Cloning, A Practical Approach,* 1, 49 (1985)], λTriplEx (manufactured by Clontech), λBlue Mid (manufactured by Clontech), λExCell (manufactured by Pharmacia), pT7T318U (manufactured by Pharmacia), pcD2 [*Mol. Cell. Biol.*, 3, 280 (1983)], pUC18 [*Gene*, 33, 103 (1985)] and the like.

Any microorganism belonging to *Escherichia coli* can be used for the host of the recombinant DNA obtained by ligating the DNA prepared in the above (1) with the vector, so long as it is a microorganism belonging to *Escherichia coli.* Examples include *Escherichia coli* XL1-Blue MRF' [manufactured by Stratagene, *Strategies,* 5, 81 (1992)], *Escherichia coli* C600 [Genetics, 39, 440 (1954)], *Escherichia coli* Y1088 [*Science*, 222, 778 (1983)], *Escherichia coli* Y1090 [*Science*, 222, 778 (1983)], *Escherichia coli* NM522 [*J. Mol. Biol.*, 166, 1 (1983)], *Escherichia coli* K802 [*J. Mol. Biol.*, 16, 118 (1966)], *Escherichia coli* JM105 [*Gene*, 38, 275 (1985)] and the like.

Any method can be used in the method for introducing the recombinant DNA, so long as it is a method for introducing DNA into the above host cell. Examples include a method using a calcium ion [*Proc. Natl. Acad. Sci. USA,* 69, 2110 (1972)], a protoplast method (Japanese Published Unexamined Patent Application No. 248394/88), electroporation *[Nucleic Acid Res.,* 16, 6127 (1988)] and the like.

The nucleotide sequence of the DNA encoding α1,4-GalNAc transferase contained in the recombinant DNA can be determined by extracting the recombinant DNA from the thus obtained transformant. For the determination of the nucleotide sequence, a conventional method, such as the dideoxy method [*Proc. Natl. Acad. Sci. USA*, 74, 5463 (1977)] or an apparatus for nucleotide sequence analysis, such as DNA Sequencer 373A (manufactured by Perkin-Elmer) or the like, can be used.

Examples of transformant containing the thus obtained recombinant DNA include *Escherichia coli* NM522/pCJ2 containing a plasmid DNA comprising the nucleotide sequence represented by SEQ ID NO:2.

### [2] Production of a protein having α1,4-GalNAc transferase activity

The protein having α1,4-GalNAc transferase activity can be produced by expressing the DNA obtained above in a host cell, for example, as shown below, using a method described in *Molecular Cloning,* Second Edition; *Current Protocols in Molecular Biology* or the like.

Specifically, based on the DNA obtained above, a DNA fragment of an appropriate length containing a portion which encodes the protein can be prepared, if necessary, and a recombinant DNA is constructed by inserting the DNA fragment into the downstream of the promoter in an appropriate expression vector. A protein having α1,4-GalNAc transferase activity can be produced by introducing the recombinant DNA into a host cell suitable for the expression vector to obtain a transformant, and culturing the transformant in a medium to accumulate the protein in the culture.

Any bacteria, yeasts, animal cells, insect cells, plant cells, and the like can be used as the host cell, so long as it can express the desired gene.

Examples of the expression vector include those which can replicate autonomously in the above-described host cell or can be integrated into chromosome and have a promoter at such a position that the DNA of encoding a protein having α1,4-GalNAc transferase activity can be transcribed.

When a procaryote, such as a bacterium or the like, is used as the host cell, it is preferred that the recombinant DNA containing the DNA encoding a protein having α1,4-GalNAc transferase activity can replicate autonomously in the procaryote and, at the same time, the recombinant vector contains a promoter, a ribosome binding sequence, the DNA of the present invention and a transcription termination sequence. A gene regulating the promoter may also be contained in the vector.

Examples of the expression vector include pBTrp2, pBTac1 and pBTac2 (all available from Boehringer Manheim), pKK223-3 (manufactured by Pharmacia), pSE280 (manufactured by Invitrogen), pGEMEX-1 (manufactured by Promega), pQE-8 (manufactured by QIAGEN), pKYP10 (Japanese Published Unexamined Patent Application No. 110600/83), pKYP200 [*Agric. Biol. Chem*., 48, 669 (1984)], pLSA1 [*Agric. Biol. Chem*., 53, 277 (1989)], pGEL1 [*Proc. Natl. Acad. Sci. USA*, 82, 4306 (1985)], pBluescript II SK(-) (manufactured by Stratagene), pTrs30 [prepared from *Escherichia coli* JM109/pTrS30 (FERM BP-5407)], pTrs32 [prepared from *Escherichia coli* JM109/pTrS32 (FERM BP-5408)], pPAC31 (WO 98/12343), pGHA2 [prepared from *Escherichia coli* IGHA2 (FERM B-400), Japanese Published Unexamined Patent Application No.221091/85], pGKA2 [prepared from *Escherichia* *coli* IGKA2 (FERM BP-6798), pTerm2 (U.S. 4,686,191, U.S. 4,939,094, U.S. 5,160,735), pSupex, pUB110, pTP5, pC194, pEG400 *[J. Bacteriol.,* 172, 2392 (1990)], pGEX (manufactured by Pharmacia), pET system (manufactured by Novagen), and the like.

Any promoter can be used so long as it can function in the host cell. Examples include promoters derived from *Escherichia coli,* phage and the like, such as trp promoter (Pₜᵣₚ), lac promoter, P_{L} promoter, P_{R} promoter, T7 promoter, and the like. Also, artificially designed and modified promoters, such as a promoter in which two Pₜᵣₚ are linked in tandem (Pₜᵣₚ×2), tac promoter, lacT7 promoter letI promoter and the like, can be used.

It is preferred to use a plasmid in which the space between Shine-Dalgarno sequence, which is the ribosome binding sequence, and the initiation codon is adjusted to an appropriate distance (for example, 6 to 18 bases).

The transcription termination sequence is not always necessary for the recombinant DNA of the present invention. However, it is preferred to provide a transcription termination sequence just downstream of the structural gene.

Examples of the host cell include microorganisms belonging to the genera *Escherichia, Serratia, Bacillus, Brevibacterium, Corynebacterium, Microbacterium, Pseudomonas,* and the like. Specific examples include *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Escherichia coli* GI698, *Escherichia coli* TB1, *Serratia ficaria, Serratia fonticola, Serratia liquefaciens, Serratia marcescens, Bacillus subtilis, Bacillus amyloliquefaciens, Corynebacterium ammoniagenes, Brevibacterium immariophilum* ATCC 14068, *Brevibacterium saccharolyticum* ATCC 14066, *Brevibacterium flavum* ATCC 14067, *Brevibacterium lactofermentum ATCC 13869, Corynebacterium glutamicum ATCC* 13032, *Corynebacterium glutamicum* ATCC 13869, *Corynebacterium acetoacidophilum* ATCC 13870, *Microbacterium ammoniaphilum* ATCC 15354, *Pseudomonas putida, Pseudomonas* sp. D-0110 and the like.

As the method for the introduction of the recombinant DNA, any method for introducing DNA into the above-described host cells, such as a method using a calcium ion [*Proc. Natl. Acad. Sci. USA,* 69, 2110 (1972)], a protoplast method (Japanese Published Unexamined Patent Application No. 248394/88), methods described in *Gene*, 17, 107 (1982) or *Mol. Gen. Genet.,* 168, 111 (1979) and the like can be used.

When yeast is used as the host cell, examples of the expression vector include YEp13 (ATCC 37115), YEp24 (ATCC 37051), YCp50 (ATCC 37419), pHS19, pHS15 and the like.

Any promoter can be used so long as it can function in a yeast strain. Examples include a promoter of a gene in glycolytic pathway such as hexose kinase *etc*., PHO5 promoter, PGK promoter, GAP promoter, ADH promoter, gal 1 promoter, gal 10 promoter, a heat shock polypeptide promoter, MFα1 promoter, CUP 1 promoter and the like.

Examples of the host cell include microorganisms belonging to the genera *Saccharomyces, Schizosaccharomyces, Kluyveromyces, Trichosporon, Schwanniomyces, Pichia, Candida* and the like. Specific examples include *Saccharomyces cerevisiae, Schlzosaccharomyces pombe, Kluyveromyces lactis, Trichosporon pullulans, Schwanniomyces alluvius, Candida utilis* and the like.

As the method for the introduction of the recombinant DNA, any method for introducing DNA into yeast, such as electroporation [*Methods. Enzymol.*, 194, 182 (1990)], a spheroplast method [*Proc. Natl. Acad. Sci. USA,* 75, 1929 (1978)], a lithium acetate method (*J. Bacteriol.,* 153, 163 (1983)] a method described in *Proc. Natl. Acad. Sci. USA*, 75, 1929 (1978) and the like can be used.

When an animal cell is used as the host cell, examples of the expression vector include pcDNAI and pcDM8 (manufactured by Funakoshi), pAGE107 [Japanese Published Unexamined Patent Application No. 22979/91, *Cytotechnology,* 3, 133 (1990)], pAS3-3 (Japanese Published Unexamined Patent Application No. 227075/90), pcDM8 [Nature, 329, 840 (1987)], pcDNAI/Amp (manufactured by Invitrogen), pREP4 (manufactured by Invitrogen), pAGE103 [*J. Biochem.*, 101, 1307 (1987)], pAGE210 and the like.

Any promoter can be used, so long as it can function in an animal cell. Examples include a promoter of IE (immediate early) gene of cytomegalovirus (CMV), an early promoter of SV40, a promoter of retrovirus, a metallothionein promoter, a heat shock promoter, SRα promoter and the like. Also, the enhancer of the IE gene of human CMV can be used together with the promoter.

Examples of the host cell include human Namalwa cell, monkey COS cell, Chinese hamster CHO cell, HBT5637 (Japanese Published Unexamined Patent Application No. 299/88) and the like.

The method for introduction of the recombinant DNA into an animal cell is not particularly limited, so long as it is a method for introducing DNA into an animal cell. Examples include electroporation [*Cytotechnology*, 3, 133 (1990)], a calcium phosphate method (Japanese Published Unexamined Patent Application No. 227075/90), lipofection [*Proc. Natl. Acad. Sci. USA*, 84, 7413 (1987)], a method described in *Virology,* 52, 456 (1973) and the like.

When an insect cell is used as the host cell, the protein can be expressed by a known method described in, for example, *Current Protocols in Molecular Biology*; *Baculovirus Expression Vectors, A Laboratory Manual,* W.H. Freeman and Company (1992); *Bio*/*Technology*, 6, 47 (1988) or the like.

Specifically, a transfer vector and baculovirus are co-transfected into an insect cell to obtain a recombinant virus in a supernatant of the culture of its insect cell, and then an insect cell is infected with the recombinant virus to express the protein.

Examples of the transfer vector used in the method include pVL1392, pVL1393 and pBlueBacIII (all manufactured by Invitrogen) and the like.

Examples of the baculovirus include *Autographa californica* nuclear polyhedrosis virus which infects insects of the family Barathra and the like.

Examples of the insect cell include *Spodoptera frugiperda* ovary cells Sf9 and Sf21 [*Baculovirus Expression Vectors, A Laboratory Manual*, W.H. Freeman and Company (1992)], *Trichoplusia ni* ovary cell High 5 (manufactured by Invitrogen) and the like.

The method for co-transfecting the transfer vector and the baculovirus for the preparation of the recombinant virus includes a calcium phosphate method (Japanese Published Unexamined Patent Application No. 227075/90), lipofection [*Proc. Natl. Acad. Sci. USA*, 84, 7413 (1987)), and the like.

When a plant cell is used as the host cell, examples of the expression vector include Ti plasmid, a tobacco mosaic virus vector and the like.

Any promoter can be used so long as it can function in a plant cell. Examples include 35S promoter of cauliflower mosaic virus (CaMV), rice actin 1 promoter and the like.

Examples of the host cell include plant cells such as tobacco, potato, tomato, carrot, soybean, rape, alfalfa, rice, wheat, barley, etc., and the like.

The method for introducing the recombinant DNA is not particularly limited, so long as it is a method for introducing DNA into a plant cell, such as the *Agrobacterium* method (Japanese Published Unexamined Patent Application No. 140885/84, Japanese Published Unexamined Patent Application No. 70080/85, WO 94/00977), electroporation (Japanese Published Unexamined Patent Application No. 251887/85), the particle gun method (Japanese Patents 2606856 and 2517813) and the like.

The protein of the present invention can be produced by culturing the thus obtained transformant of the present invention in a medium to form and accumulate the protein in the culture, and recovering the protein from the culture. Culturing of the transformant of the present invention in a medium is carried out according to the usual method as used in culturing of the host.

As a medium for culturing the transformant of the present invention obtained by using, as the host, prokaryote such as *Escherichia coli* or the like or eukaryote such as yeast or the like, the medium may be either a natural medium or a synthetic medium, so long as it contains a carbon source, a nitrogen source, inorganic salts and the like which can be assimilated by the organism and the transformant can be cultured efficiently.

Any carbon source which can be assimilated by the transformant is used. Examples include carbohydrates such as glucose, fructose, sucrose, molasses containing them, starch, starch hydrolysate, *etc.*, organic acids such as acetic acid, propionic acid, *etc.*, alcohols such as ethanol, propanol, *etc.*, and the like.

Examples of the nitrogen source include ammonia, various ammonium salts of inorganic acids or organic acids such as ammonium chloride, ammonium sulfate, ammonium acetate, ammonium phosphate, *etc.*, other nitrogen-containing compounds, peptone, meat extract, yeast extract, corn steep liquor, casein hydrolysate, soybean meal and soybean meal hydrolysate, various fermented cells and hydrolysates thereof, and the like.

Examples of the inorganic salts include potassium dihydrogen phosphate, dipotassium hydrogen phosphate, magnesium phosphate, magnesium sulfate, sodium chloride, ferrous sulfate, manganese sulfate, copper sulfate, calcium carbonate and the like.

Culturing is usually carried out under aerobic conditions by shaking culture, submerged spinner culture under aeration or the like. The temperature of culturing is preferably from 15 to 40°C, and the culturing time is generally from 16 hours to 7 days. The pH of the medium is preferably maintained at 3.0 to 9.0 during culturing. The pH can be adjusted using an inorganic or organic acid, an alkali solution, urea, calcium carbonate, ammonia or the like.

Also, antibiotics such as ampicillin, tetracycline, chloramphenicol and the like can be added to the medium during culturing, if necessary.

When a microorganism transformed with an expression vector containing an inducible promoter is cultured, an inducer can be added to the medium, if necessary. For example, isopropyl-β-D-thiogalactopyranoside (IPTG) or the like can be added to the medium when a microorganism transformed with an expression vector containing *lac* promoter is cultured, or indoleacrylic acid or the like can by added thereto when a microorganism transformed with an expression vector containing *trp* promoter is cultured.

Examples of the medium for culturing a transformant obtained using an animal cell as the host include generally used RPMI 1640 medium [*J. Am. Med. Assoc.*, 199, 519 (1967)], Eagle's MEM *[Science,* 122, 501 (1952)], modified Dulbecco's MEM [*Virology*, 8, 396 (1959)], and 199 Medium [*Proc. Soc. Biol. Med*., 73, 1 (1950)], and other media to which fetal calf serum or the like has been added to the above media and the like.

Culturing is generally carried out at pH 6 to 8 and at 30 to 40°C for 1 to 7 days in the presence of 5% CO₂.

Furthermore, if necessary, antibiotics such as kanamycin, penicillin and the like may be added to the medium during culturing.

Examples of the medium for culturing a transformant obtained using an insect cell as the host include generally used TNM-FH medium (manufactured by Pharmingen), Sf-900 II SFM (manufactured by Life Technologies), ExCell 400 and ExCell 405 (both manufactured by JRH Biosciences), Grace's Insect Medium *[Nature,* 195, 788 (1962)] and the like.

Culturing is generally carried out at pH 6 to 7 and at 25 to 30°C for 1 to 5 days.

Furthermore, if necessary, antibiotics such as gentamicin and the like may be added to the medium during culturing.

A transformant obtained using a plant cell as the host cell can be used as the cell or after differentiating to a plant cell or organ. Examples of the medium used in culturing of the transformant include Murashige and Skoog (MS) medium, White medium, media to which a plant hormone such as auxin, cytokinine or the like has been added, and the like.

Culturing is carried out generally at a pH 5 to 9 and at 20 to 40°C for 3 to 60 days.

Furthermore, if necessary, antibiotics such as kanamycin, hygromycin and the like can be added to the medium during culturing.

As described above, the protein can be produced by culturing a transformant derived from a microorganism, animal cell or plant cell containing a recombinant DNA to which a DNA encoding the polypeptide of the present invention has been inserted, according to the usual culturing method to form and accumulate the protein, and recovering the protein from the culture.

The process for producing the protein of the present invention includes a method of intracellular production in a host cell, a method of extracellular secretion from a host cell, or a method of production on an outer membrane of the host cell. The method can be selected by changing the host cell employed or the structure of the protein produced.

When the protein of the present invention is produced in a host cell or on an outer membrane of the host cell, the protein can be actively secreted extracellularly according to, for example, the method of Paulson *et al*. [*J. Biol. Chem.*, 264, 17619 (1989)], the method of Lowe *et al.* [*Proc. Natl. Acad. Sci. USA*, 86, 8227 (1989); *Genes Develop.,* 4, 1288 (1990)], or methods described in Japanese Published Unexamined Patent Application Nos. 336963/93, 823021/94, and the like.

Specifically, the protein of the present invention can be actively secreted extracellularly by expressing it in the form that a signal peptide has been added to the N-terminal side of a polypeptide containing an active site of the protein of the present invention according to the recombinant DNA technique.

Furthermore, the amount produced can be increased using a gene amplification system, such as by use of a dihydrofolate reductase gene or the like according to the method described in Japanese Published Unexamined Patent Application No. 227075/90.

Moreover, the protein of the present invention can be produced by redifferentiating animal or plant cells to which the gene has been introduced to prepare a gene-introduced animal individual (transgenic nonhuman animal) or plant individual (transgenic plant) and using the individuals.

When the transformant is the animal individual or plant individual, the protein of the present invention can be produced by breeding or cultivating it so as to form and accumulate the protein, and recovering the protein from the animal individual or plant individual.

Examples of the process for producing the protein of the present invention using the animal individual include a process for producing the protein of the present invention in an animal developed by introducing a gene according to known methods [*Am. J. Clin. Nutr.*, 63, 639S (1996), *Am. J. Clin. Nutr.*, 63, 627S (1996), *Bio*/*Technology*, 9, 830 (1991)].

In the case of the animal individual, the protein can be produced by breeding a transgenic nonhuman animal to which the DNA encoding the protein of the present invention has been introduced to form and accumulate the protein in the animal, and recovering the protein from the animal. Examples of the production and accumulation place in the animal include milk (Japanese Published Unexamined Patent Application No. 309192/88), egg and the like of the animal. Any promoter can be used, so long as it can function in the animal. Suitable examples include an α-casein promoter, a β-casein promoter, a β-lactoglobulin promoter, a whey acidic protein promoter, and the like, which are specific for mammary glandular cells.

Examples of the process for producing the protein of the present invention using the plant individual include a process for producing the protein of the present invention by cultivating a transgenic plant, to which the DNA encoding the protein of the present invention is introduced, by a known method [*Tissue Culture (Soshiki Baiyo*), *20* (1994), *Tissue Culture (Soshiki Baiyo), 21* (1994), *Trends Biotechnol.*, 15, 45 (1997)] to form and accumulate the protein in the plant, and recovering the protein from the plant.

The protein produced by the transformant of the present invention can be isolated and purified using the general method for isolating and purifying an enzyme. For example, when the protein of the present invention is expressed as a soluble product in the host cells, the cells are collected by centrifugation after culturing, suspended in an aqueous buffer, and disrupted using an ultrasonicator, a French press, a Manton Gaulin homogenizer, a Dynomill, or the like to obtain a cell-free extract. From the supernatant obtained by centrifuging the cell-free extract, a purified product can be obtained by the usual method used for isolating and purifying an enzyme, for example, solvent extraction, salting-out using ammonium sulfate or the like, desalting, precipitation using an organic solvent, anion exchange chromatography using a resin, such as diethylaminoethyl (DEAE)-Sepharose, DIAION HPA-75 (manufactured by Mitsubishi Chemical) or the like, cation exchange chromatography using a resin, such as S-Sepharose FF (manufactured by Pharmacia) or the like, hydrophobic chromatography using a resin, such as butyl sepharose, phenyl sepharose or the like, gel filtration using a molecular sieve, affinity chromatography, chromatofocusing, or electrophoresis, such as isoelectronic focusing or the like, alone or in combination thereof.

When the protein is expressed as an inclusion body in the host cells, the cells are collected in the same manner, disrupted and centrifuged to recover the protein as the precipitate fraction. Next, the inclusion body of the protein is solubilized with a protein-denaturing agent. The solubilized protein solution is diluted or dialyzed to lower the concentration of the protein denaturing agent in the solution. Thus, the normal tertiary structure of the protein is reconstituted. After the procedure, a purified product of the protein can be obtained by a purification/isolation method similar to the above.

When the protein of the present invention or its derivatives of the protein in which a sugar chain is added to the protein are secreted out of cells, the protein or the derivatives can be collected in the culture supernatant. Specifically, the culture supernatant is obtained by treating the culture in a treatment similar to the above, such as centrifugation or the like. Then a purified product can be obtained from the supernatant using a purification/isolation method similar to the above.

Examples of the protein obtained by the above method include a protein comprising the amino acid sequence represented by SEQ ID NO:1.

Also, the protein of the present invention can be produced by a chemical synthesis method, such as Fmoc (fluorenylmethyloxycarbonyl) method, tBoc (t-butyloxycarbonyl) method or the like. It can also be chemically synthesized using a peptide synthesizer manufactured by Advanced ChemTech, Perkin-Elmer, Pharmacia, Protein Technology Instrument, Synthecell-Vega, PerSeptive, Shimadzu Corporation or the like.

### [3] Preparation of GalNAc-containing complex carbohydrate

A GalNAc-containing complex carbohydrate can be produced in an aqueous medium using a culture of the transformant obtained by the culturing described in [2] or a treated product of the culture as the enzyme source.

Examples of the treated product of the culture include a concentrated product of the culture, a dried product of the culture, cells obtained by centrifuging the culture, a dried product of the cells, a freeze-dried product of the cells, a surfactant-treated product of the cells, an ultrasonic-treated product of the cells, a mechanically ground product of the cells, a solvent-treated product of the cells, an enzyme-treated product of the cells, a protein fraction of the cells, an immobilized product of the cells and an enzyme preparation obtained by extracting from the cells.

The enzyme source to be used in the formation of GalNAc-containing complex carbohydrate is used at a concentration of 0.1 mU/L to 10,000 U/L, preferably 1 mU/L to 1,000 U/L, when the activity capable of forming 1 µmol of GalNAc-containing complex carbohydrate at 37°C in one minute is defined as 1 unit (U).

Examples of the aqueous medium used in the preparation of GalNAc-containing complex carbohydrate include water, buffers of phosphate, carbonate, acetate, borate, citrate, tris, *etc.*, alcohol such as methanol, ethanol, *etc.*, esters such as ethyl acetate, *etc.*, ketones such as acetone, *etc.*, amides such as acetamide, etc. and the like. Also, the microbial culture used as the enzyme source can be used as an aqueous medium.

In preparing GalNAc-containing complex carbohydrate, a surfactant or an organic solvent may be added, if necessary. Any compound capable of accelerating preparation of GalNAc-containing complex carbohydrate may be used as the surfactant. Examples include nonionic surfactants such as polyoxyethylene octadecylamine (e.g., Nymeen S-215, manufactured by Nippon Oil & Fats), etc.; cationic surfactants such as cetyltrimethylammonium bromide, alkyldimethyl benzylammoniumchloride (e.g., Cation F2-40E, manufactured by Nippon Oil & Fats), *etc.*; anionic surfactants such as lauroyl sarcosinate, etc.; tertiary amines such as alkyldimethylamine (e.g., Tertiary Amine FB, manufactured by Nippon Oil & Fats), *etc.*; and the like, which may be used alone or as a mixture of two or more. The surfactant is used generally at a concentration of 0.1 to 50 g/L. Examples of the organic solvent include xylene, toluene, a fatty acid alcohol, acetone, ethyl acetate and the like, which are generally used at a concentration of 0.1 to 50 ml/L.

Regarding UDP-GalNAc as the sugar nucleotide substrate used in the preparation of GalNAc-containing complex carbohydrate, a reaction solution containing UDP-GalNAc obtained by using activity such as a microorganism or the like or UDP-GalNAc purified from the reaction solution can be used, in addition to a commercial product.

The sugar nucleotide substrate is used at a concentration of 0.1 to 500 mmol/L.

As the acceptor complex carbohydrate used in the formation of GalNAc-containing complex carbohydrate, any substance which can become a substrate of the transferase can be used. Examples include lactose and lacto-N-neotetraose, as well as an oligosaccharide consisting of 10 or less sugar having a lactose, N-acetyllactosamine, lacto-N-neotetraose, lacto-N-tetraose, paralacto-N-neohexaose, Le^{x} or Le^{a} structure in the non-reducing terminal or the like.

The acceptor complex carbohydrate is used at a concentration of 0.1 to 500 mmol/L.

In the production reaction, inorganic salts such as MgCl₂, *etc*., β-mercaptoethanol and the like can be added, if necessary.

The GalNAc-containing complex carbohydrate formation reaction is carried out in an aqueous medium at pH 5 to 10, preferably pH 6 to 8, and at 20 to 50°C for 1 to 96 hours.

The amount of GalNAc-containing complex carbohydrate produced in the aqueous medium can be determined in accordance with a known method [*Chemistry and Industry* (*Kagaku to Kogyo*), 43, 953 (1990)].

The GalNAc-containing complex carbohydrate produced in the reaction solution can be recovered by the usual method using activated charcoal, an ion exchange resin or the like.

### BRIEF DESCRIPTION OF THE DRAWING

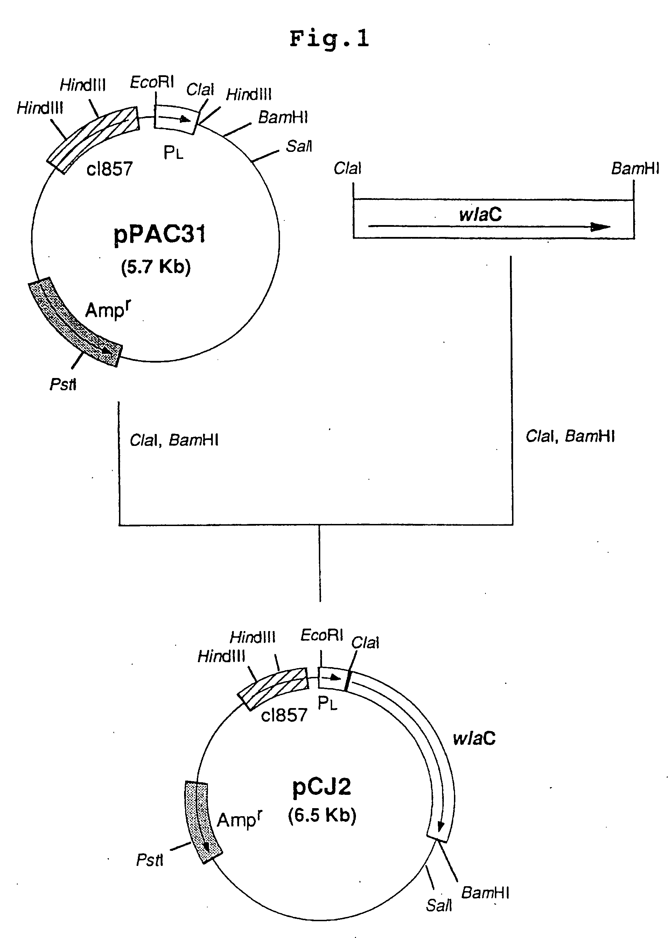

Fig. 1 is a schematic illustration showing structure of an α1,4-GalNAc transferase gene expression plasmid pCJ2.

The reference signs in Fig. 1 have the following meanings.
- Ampr:: ampicillin resistance gene
- P_{L}:: P_{L} prompter
- cI867:: cI867 repressor
- wlaC:: α1,4-GalNAc transferase gene

### BEST MODE FOR CARRYING OUT THE INVENTION

Examples of the present invention are described below, but the present invention is not limited to the examples.

### Example 1

### Construction of transformant expressing α1,4-GalNAc transferase:

*Campylobacter jejuni* (ATCC 43446) was cultured by a known method *[Microbiology,* 144, 2049 (1998)]. After culturing, chromosomal DNA of the microorganism was isolated and purified by the method described in *Current Protocols in Molecular Biology.*

DNAs comprising the nucleotide sequences represented by SEQ ID NOs:3 and 4 were synthesized using Model 8905 DNA Synthesizer manufactured by Perceptive Biosystems.

PCR was carried out using the above synthetic DNAs as primer sets and the *Campylobacter jejuni* (ATCC 43446) chromosomal DNA as the template. Using 40 µl of a reaction solution containing 0.1 µg of the chromosomal DNA, 0.5 µmol/L of each primer, 2.5 units of Pfu DNA polymerase (manufactured by Stratagene), 4 µl of 10× buffer for Pfu DNA polymerase (manufactured by Stratagene) and 200 µmol/L of each deoxy NTP, PCR was carried out by 30 cycles, one cycle consisting of a reaction at 94°C for 1 minute, reaction at 37°C for 2 minutes and reaction at 72°C for 3 minutes.

After confirming that the desired fragment was amplified by subjecting 1/10 volume of the reaction solution to agarose gel electrophoresis, the remaining reaction solution was mixed with the same volume of TE [10 mmol/L Tris-HCl (pH 8.0), 1 mmol/L EDTA] saturated phenol/chloroform (1 vol/1 vol).

The mixed solution was centrifuged, and then the thus obtained upper layer was mixed with 2 volumes of cold ethanol and allowed to stand at -80°C for 30 minutes. The resulting solution was centrifuged to obtain a DNA precipitate.

The DNA precipitate was dissolved in 20 µl of TE. Using 5 µl of the dissolved solution, the DNA was digested with restriction enzymes *Cla*I and *Bam*HI and then, using a ligation kit (manufactured by Takara Shuzo), subjected to the ligation at 16°C for 16 hours together with 0.2 µg of pPAC31 DNA digested with restriction enzymes *Cla*I and *Bam*HI.

*Escherichia coli* NM522 was transformed with the ligation solution in accordance with the above known method, and the transformants were spread on LB agar medium [10 g/L bacto tryptone (manufactured by Difco), 5 g/L yeast extract (manufactured by Difco), 5 g/L NaCl (pH 7.2), 15 g/L agar] containing 50 µg/ml ampicillin, followed by culturing overnight at 30°C.

A plasmid was extracted from a thus grown transformant colony in accordance with the known method, and it was confirmed that the plasmid is the desired plasmid pCJ2 by analyzing restriction enzyme sites and the like of the plasmid.

Based on the above, it was confirmed that a transformant *Escherichia coli* NM522/pCJ2 expressing α1,4-GalNAc transferase gene was obtained.

### Example 2

### Production of GalNAcα1-4Galβ1-4GlcNAcβ1-3Galβ1-4Glc:

*Escherichia coli* NM522/pCJ2 obtained in Example 1 was inoculated into 8 ml of LB medium containing 50 µg/ml ampicillin in a large test tube and cultured at 28°C for 17 hours. The resulting culture was inoculated into 400 ml of LB medium containing 50 µg/ml ampicillin in a 2-L Erlenmeyer flask with baffles in an amount of 1% and cultured at 30°C for 4 hours, followed by culturing at 40°C for 3 hours. The resulting culture was centrifuged to obtain wet cells. The wet cells could be stored at -20°C, if necessary, and it was able to use them by thawing before to use.

A reaction solution of 4 ml composed of 100 g/L of the wet cells, 50 mmol/L citrate buffer (pH 7.0), 10 mmol/L MnCl₂, 40 mmol/L lacto-N-neotetraose, 4 mmol/L UDP-GalNAc and 0.4% Nymeen S-215 was prepared, and the reaction was carried out at 37°C for 24 hours.

After completion of the reaction, the reaction product was analyzed using a carbohydrate analysis system (DX-500) manufactured by Dionex under the following analyzing conditions to confirm that a new oligosaccharide (NOS-α) was formed and accumulated in the reaction solution. Analyzing conditions:
- Column:: CarboPAC PA10
- Eluent:: A; H₂O, B; 500 mmol/L NaOH
- Gradient:: 8 to 20% B in 21 min
- Detector:: Pulsed amperometry detector

The reaction solution was heat-treated at 100°C for 2 minutes, and then the cells were removed by centrifugation. The resulting supernatant was applied to a column (2.5 cm in inner diameter, 120 cm in length) packed with BioGel-P2 (manufactured by Bio-Rad) and then eluted with H₂O. Fractions containing NOS-α were combined and concentrated using an evaporator.

Molecular weight of the thus isolated NOS-α was measured by a mass spectrometer JMS-HX100/110A manufactured by JEOL LTD., and then it was shown that molecular weight of NOS-α is 910. Also, since the following signals were obtained as main signals as a result of NMR analysis of NOS-α by JNM-A400 manufactured by JEOL LTD., it was revealed that the structure of NOS-α is GalNAcα1-4Galβ1-4GlcNAcβ1-3Galβ1-4Glc.
¹H: 2.07, 2.10, 3.31, 3.61, 3.62, 3.62, 3.66, 3.66, 3.67, 3.76, 3.77, 3.77, 3.77, 3.78, 3.79, 3.83, 3.86, 4.00, 4.03, 4.08, 4.19, 4.23, 4.42, 4.47, 4.57, 4.70, 4.75, 4.93, 5.26 ¹³C: 22.9, 22.9, 50.8, 56.2, 61.3, 67.9, 68.9, 69.1, 70.7, 71.5, 71.6, 71.9, 72.2, 72.9, 72.9, 74.6, 75.1, 77.4, 79.2, 79.2, 79.2, 82.8, 92.5, 96.5, 99.1, 103.4, 103.7, 103.9, 175.2, 175.7

### Example 3

### Production of GalNAcal-4Galβ1-4Glc:

*Escherichia coli* NM522/pCJ2 obtained in Example 1 was inoculated into 8 ml of LB medium containing 50 µg/ml ampicillin in a large test tube and cultured at 28°C for 17 hours. The resulting culture was inoculated into 400 ml of LB medium containing 50 µg/ml of ampicillin in a 2-L Erlenmeyer flask with baffles in an amount of 1% and cultured at 30°C for 4 hours, followed by culturing at 40°C for 3 hours. The resulting culture was centrifuged to obtain wet cells. The wet cells could be stored at -20°C, if necessary, and it was able to use them by thawing before to use.

A reaction solution of 4 ml composed of 100 g/L of the wet cells, 50 mmol/L citrate buffer (pH 7.0), 10 mmol/L MnCl₂, 40 mmol/L lactose, 4 mmol/L UDP-GalNAc and 0.4% Nymeen S-215 was prepared, and the reaction was carried out at 37°C for 24 hours.

After completion of the reaction, the reaction product was analyzed using a carbohydrate analysis system (DX-500) manufactured by Dionex (under the same analyzing conditions described in Example 2) to confirm that a new oligosaccharide (NOS-β) was formed and accumulated in the reaction solution.

The reaction solution was heat-treated at 100°C for 2 minutes, and then the cells were removed by centrifugation. The resulting supernatant was applied to a column (2.5 cm in inner diameter, 120 cm in length) packed with BioGel-P2 (manufactured by Bio-Rad) and then eluted with H₂O. Fractions containing NOS-β were combined and concentrated using an evaporator.

The structure of the thus isolated NOS-β was analyzed by NMR, it was revealed that the structure of NOS-β is GalNAcα1-4Galβ1-4Glc.

### INDUSTRIAL APPLICABILITY

According to the present invention, α1,4-GalNAc transferase can be produced in a large amount by recombinant DNA techniques. Also, GalNAc-containing complex carbohydrates can be produced efficiently by using the enzyme.

### FREE TEXT OF SEQUENCE LISTING

SEQ ID NO:3 - Explanation of synthetic sequence: Synthetic DNA
SEQ ID NO:4 - Explanation of synthetic sequence: Synthetic DNA

## Claims

1. A process for producing a complex carbohydrate containing N-acetylgalactosamine (hereinafter referred to as "GalNAc"), which comprises selecting, as an enzyme source, a culture of a transformant expressing a protein having α1,4-N-acetylgalactosamine transferase (hereinafter referred to as "α1,4-GalNAc transferase") activity derived from a microorganism or a treated product of the culture; allowing the enzyme source, an acceptor complex carbohydrate and uridine diphospho-N-acetylgalactosamine (hereinafter referred to as "UDP-GalNAc") to be present in an aqueous medium; transferring GalNAc to the acceptor complex carbohydrate to form and accumulate the GalNAc-containing complex carbohydrate in the aqueous medium; and recovering the GalNAc-containing complex carbohydrate from the aqueous medium.

2. A process for producing a protein having α1,4-GalNAc transferase activity, which comprises culturing a transformant expressing a protein having α1,4-GalNAc transferase activity derived from a microorganism in a medium; allowing the protein having α1,4-GalNAc transferase activity to form and accumulate in the culture; and recovering the protein from the culture.

3. The process according to claim 1, wherein the GalNAc-containing complex carbohydrate is NOS-α having a GalNAcα1-4Galβ1-4GlcNAcβ-1-3Galβ1-4Glc structure or NOS-β having a GalNAcα1-4Galβ1-4Glc structure.

4. The process according to claim 1 or 2, wherein the protein having α1,4-GalNAc transferase activity derived from a microorganism is a protein selected from proteins described in the following (1) to (3):
(1) a protein having the transferase activity derived from a microorganism belonging to the genus *Campylobacter*;
(2) a protein comprising the amino acid sequence represented by SEQ ID NO:1;
(3) a protein which comprises an amino acid sequence in which at least one amino acid in the amino acid sequence represented by SEQ ID NO:1 is deleted, substituted or added and has the transferase activity.

5. The process according to claim 1 or 2, wherein the transformant is a transformant obtained by introducing a recombinant DNA into a microorganism.

6. The process according to claim 5, wherein the microorganism is a microorganism belonging to the genus *Escherichia.*

7. The process according to claim 6, wherein the microorganism belonging to the genus *Escherichia* is *Escherichia coli*.

8. The process according to claim 5, wherein the recombinant DNA is a recombinant DNA comprising a DNA selected from DNAs described in the following (1) to (6):
(1) a DNA encoding a protein having α1,4-GalNAc transferase activity derived from a microorganism;
(2) a DNA encoding a protein having α1,4-GalNAc transferase activity derived from a microorganism belonging to the genus *Campylobacter*;
(3) a DNA encoding a protein comprising the amino acid sequence represented by SEQ ID NO:1;
(4) a DNA comprising the nucleotide sequence represented by SEQ ID NO:2;
(5) a DNA encoding a protein which comprises an amino acid sequence in which at least one amino acid in the amino acid sequence represented by SEQ ID NO:1 is deleted, substituted or added, and has α1,4-GalNAc transferase activity;
(6) a DNA which hybridizes with the DNA of any one of above (1) to (5) under stringent conditions and encodes a protein having α1,4-GalNAc transferase activity.

9. The process for producing GalNAc-containing complex carbohydrate according to claim 1, wherein the acceptor complex carbohydrate is a complex carbohydrate which contains an oligosaccharide consisting of 10 or less sugar having galactose or GalNAc in the non-reducing terminal.

10. The process according to claim 9, wherein the oligosaccharide has a lactose, N-acetyllactosamine, lacto-N-neotetraose, lacto-N-tetraose, paralacto-N-neohexaose, Le^{x} or Le^{a} structure in the non-reducing terminal.

11. The process for producing GalNAc-containing complex carbohydrate according to claim 1, wherein the treated product of the culture is a concentrated product of the culture, a dried product of the culture, cells obtained by centrifuging the culture, a dried product of the cells, a freeze-dried product of the cells, a surfactant-treated product of the cells, an ultrasonic-treated product of the cells, a mechanically ground product of the cells, a solvent-treated product of the cells, an enzyme-treated product of the cells, a protein fraction of the cells, an immobilized product of the cells or an enzyme preparation obtained by extracting the cells.

12. A GalNAc-containing complex carbohydrate according to the following (1) or (2):
(1) NOS-α having a GalNAcα1-4Galβ1-4GlcNAcβ-1-3Galβ1-4Glc structure;
(2) NOS-β having a GalNAcα1-4Galβ1-4Glc structure.

13. An agent for synthesizing a GalNAc-containing complex carbohydrate, which contains a protein comprising the amino acid sequence represented by SEQ ID NO:1.

14. A protein which comprises an amino acid sequence in which at least one amino acid in the amino acid sequence represented by SEQ ID NO:1 is deleted, substituted or added, and has α1,4-GalNAc transferase activity.

15. A DNA according to any one of the following
(1) to (3):
(1) a DNA encoding the protein according to claim 14;
(2) a DNA comprising a nucleotide sequence in which at least one base in the nucleotide sequence represented by SEQ ID NO:2 is deleted, substituted or added, which is a DNA encoding a protein having α1,4-GalNAc transferase activity;
(3) a DNA which hybridizes with any one of DNAs selected from the DNA of (1), the DNA of (2) and a DNA comprising the nucleotide sequence represented by SEQ ID NO:2 under stringent conditions, and encodes a protein having α1,4-GalNAc transferase activity.

16. A recombinant DNA which is obtained by ligating the DNA according to claim 15 with a vector.

17. A transformant which is obtained by introducing the recombinant DNA according to claim 16 into a host cell.

18. The transformant according to claim 17, wherein the host cell is a microorganism.

19. The transformant according to claim 18, wherein the microorganism is a microorganism belonging to the genus *Escherichia.*

20. The transformant according to claim 19, wherein the microorganism belonging to the genus *Escherichia* is *Escherichia coli.*


SEQUENCE LISTING

<110> KYOWA HAKKO KOGYO CO.,LTD

<120> Alpha 1,4-N-acetylgalactosaminyltransferase gene and the process for producing of alpha 1,4-N-acetylgalactosaminyltransferase and complex carbohydrate containing N-acetylgalactosamine

<130> 11298W01

<140>
<141>

<150> JP 00/109150
<151> 2000-4-11

<160> 4

<170> PatentIn Ver. 2.0

<210> 1
<211> 358
<212> PRT
<213> Campylobacter jejuni (ATCC43446)

<400> 1
Met Lys Ile Ser Phe Ile Ile Ala Thr Leu Asn Ser Gly Gly Ala Glu
1               5                   10                  15

Arg Val Leu Val Thr Leu Ala Asn Ala Leu Cys Lys Glu His Glu Val
            20                  25                  30

Ser Ile Ile Lys Phe His Thr Gly Glu Ser Phe Tyr Lys Leu Glu Asn
        35                  40                  45

Glu Val Lys Val Thr Ser Leu Glu Gln Phe Arg Phe Asp Thr Leu Tyr
    50                  55                  60

His Lys Ile Ala Ser Arg Phe Lys Lys Phe Phe Ala Leu Arg Lys Ala
65                  70                  75                  80

Leu Lys Glu Ser Lys Ala Asp Val Phe Ile Ser Phe Leu Asp Thr Thr
                85                  90                  95

Asn Ile Ala Cys Ile Leu Ala Asn Ile Gly Leu Lys Thr Pro Leu Ile
            100                 105                 110

Ile Ser Glu His Ser Asn Glu Ala Tyr Leu Lys Pro Lys Thr Trp Arg
        115                 120                 125

Phe Leu Arg Arg Val Ser Tyr Pro Phe Cys Asp Ala Leu Ser Val Leu
    130                 135                 140

Gly Ser Ser Asp Lys Val Tyr Tyr Glu Arg Phe Val Lys Arg Val Lys
145                 150                 155                 160

Leu Leu Leu Asn Pro Cys His Phe Ser Asp Glu Ile Pro Phe Asp Ser
                165                 170                 175

Ser Phe Glu Lys Glu Asn Leu Val Leu Phe Ile Gly Arg Leu Asp His
            180                 185                 190

Met Lys Asn Pro Val Met Phe Leu Lys Ala Ile Ala His Leu Asp Lys
        195                 200                 205

Asn Leu Gln Glu Asn Tyr Lys Phe Val Ile Ala Gly Asp Gly Glu Leu
    210                 215                 220

Arg Gln Glu Leu Glu Tyr Lys Val Lys Ser Leu Gly Ile Lys Val Asp
225                 230                 235                 240

Phe Leu Gly Arg Val Glu Asn Val Lys Ala Leu Tyr Glu Lys Ala Lys
                245                 250                 255

Val Leu Cys Leu Cys Ser Phe Val Glu Gly Leu Pro Thr Val Leu Ile
            260                 265                 270

Glu Ser Leu Tyr Phe Glu Val Cys Arg Ile Ser Ser Ser Tyr Tyr Asn
        275                 280                 285

Gly Ala Lys Asp Leu Ile Lys Asp Asn His Asp Gly Leu Leu Val Gly

290                 295                 300

Cys Asp Asp Glu Ile Ala Leu Ala Lys Lys Leu Glu Leu Val Leu Asn
305                 310                 315                 320

Asp Glu Asn Phe Arg Lys Glu Leu Val Asn Asn Ala Lys Gln Arg Cys
                325                 330                 335

Lys Asp Phe Glu Ile Ser Asn Ile Lys Glu Glu Trp Leu Lys Leu Ile
            340                 345                 350

Val Glu Val Lys Asn Ala
        355         358

<210> 2
<211> 1074
<212> DNA
<213> Campylobacter jejuni (ATCC43446)

<220>
<221> CDS
<222> (1)..(1074)

<400> 2
atg aaa ata agc ttt att atc gca act tta aat tca gga ggc gct gag      48
Met Lys Ile Ser Phe Ile Ile Ala Thr Leu Asn Ser Gly Gly Ala Glu
1               5                   10                  15

cgt gtt tta gta acc tta gct aat gcg ctt tgc aaa gag cat gaa gta      96
Arg Val Leu Val Thr Leu Ala Asn Ala Leu Cys Lys Glu His Glu Val
            20                  25                  30

agt att att aaa ttt cat aca gga gaa tct ttt tat aag ctt gaa aat      144
Ser Ile Ile Lys Phe His Thr Gly Glu Ser Phe Tyr Lys Leu Glu Asn
        35                  40                  45

gaa gtt aaa gtt aca agt tta gaa caa ttt aga ttt gac acg ctt tat      192
Glu Val Lys Val Thr Ser Leu Glu Gln Phe Arg Phe Asp Thr Leu Tyr
    50                  55                  60

cat aaa atc gca agt cgt ttt aag aaa ttt ttt gcc tta aga aag gct 240
His Lys Ile Ala Ser Arg Phe Lys Lys Phe Phe Ala Leu Arg Lys Ala
65                  70                  75                  80

tta aaa gaa agt aag gct gat gtt ttt att tct ttt ttg gat aca act 288
Leu Lys Glu Ser Lys Ala Asp Val Phe Ile Ser Phe Leu Asp Thr Thr
                85                  90                  95

aat att gct tgt att cta gct aat ata gga ctt aaa act cca ctc atc 336
Asn Ile Ala Cys Ile Leu Ala Asn Ile Gly Leu Lys Thr Pro Leu Ile
            100                 105                 110

att agc gag cat agt aat gaa gcg tat tta aaa cct aaa act tgg cgt 384
Ile Ser Glu His Ser Asn Glu Ala Tyr Leu Lys Pro Lys Thr Trp Arg
        115                 120                 125

ttt tta aga agg gta agc tat cct ttt tgt gat gct tta agt gtg ctt 432
Phe Leu Arg Arg Val Ser Tyr Pro Phe Cys Asp Ala Leu Ser Val Leu
    130                 135                 140

gga agc agt gat aag gtg tat tat gaa aga ttt gta aaa agg gtt aag 480
Gly Ser Ser Asp Lys Val Tyr Tyr Glu Arg Phe Val Lys Arg Val Lys
145                 150                 155                 160

ctt tta tta aac cct tgt cat ttt agc gat gaa att cct ttt gat tct 528
Leu Leu Leu Asn Pro Cys His Phe Ser Asp Glu Ile Pro Phe Asp Ser
                165                 170                 175

agt ttt gaa aaa gaa aat ttg gtt ctt ttt ata ggg cgt tta gat cac 576
Ser Phe Glu Lys Glu Asn Leu Val Leu Phe Ile Gly Arg Leu Asp His
            180                 185                 190

aac aaa aac cct gta atg ttt tta aaa gct ata gca cat ttg gat aaa 624
Asn Lys Asn Pro Val Met Phe Leu Lys Ala Ile Ala His Leu Asp Lys
        195                 200                 205

aat ttg caa gaa aat tat aaa ttt gtt ata gca gga gat gga gag tta 672
Asn Leu Gln Glu Asn Tyr Lys Phe Val Ile Ala Gly Asp Gly Glu Leu
    210                 215                 220

agg caa gaa ctt gaa tac aag gta aaa tct tta gga ata aaa gtt gat 720

Arg Gln Glu Leu Glu Tyr Lys Val Lys Ser Leu Gly Ile Lys Val Asp
225                 230                 235                 240

ttt tta gga cgc gtt gaa aat gtc aag gct ctt tat gaa aaa gca aaa     768
Phe Leu Gly Arg Val Glu Asn Val Lys Ala Leu Tyr Glu Lys Ala Lys
                245                 250                 255

gtg ctt tgc ctt tgt tct ttt gta gag ggt ttg cca acg gtt tta att     816
Val Leu Cys Leu Cys Ser Phe Val Glu Gly Leu Pro Thr Val Leu Ile
            260                 265                 270

gaa agt ttg tat ttt gag gtt tgt aga att tca agt tct tat tat aat     864
Glu Ser Leu Tyr Phe Glu Val Cys Arg Ile Ser Ser Ser Tyr Tyr Asn
        275                 280                 285

ggt gct aag gat tta atc aaa gat aat cat gat ggg ctt ttg gta ggt     912
Gly Ala Lys Asp Leu Ile Lys Asp Asn His Asp Gly Leu Leu Val Gly
    290                 295                 300

tgt gat gat gaa ata gca ctt gct aaa aaa ctt gaa ctt gtt tta aat     960
Cys Asp Asp Glu Ile Ala Leu Ala Lys Lys Leu Glu Leu Val Leu Asn
305                 310                 315                 320

gat gaa aa ttt tag aaa a gaa ctt gta aat aat gcc aaa caa agg tgt     1008
Asp Glu Asn Phe Arg Lys Glu Leu Val Asn Asn Ala Lys Gln Arg Cys
                325                 330                 335

aaa gac ttt gaa att tct aac atc aaa gaa gaa tgg ctt aag ctt ata     1056
Lys Asp Phe Glu Ile Ser Asn Ile Lys Glu Glu Trp Leu Lys Leu Ile
            340                 345                 350

gtc gag gtt aaa aat gcc                                             1074
Val Glu Val Lys Asn Ala
        355         358

<210> 3
<211> 29
<212> DNA
<213> Artificial Sequence

<220>
<223> Description of Artificial Sequence: Synthetic DNA
<400> 3
aggaatcgat atgaaaataa gctttatta                                    29

<210> 4
<211> 29
<212> DNA
<213> Artificial Sequence

<220>
<223> Description of Artificial Sequence: Synthetic DNA

<400> 4
agttggatcc ataacagaaa gtttaggca                                    29